# EUROPÄISCHE PATENTSCHRIFT

(11) **EP 1 208 808 B1**
(45) Veröffentlichungstag und Bekanntmachung des Hinweises auf die Patenterteilung: **18.06.2003**
(21) Anmeldenummer: 00125274.1
(22) Anmeldetag: 24.11.2000
(51) Int. Cl.: A61B 19/00, G06F 19/00

(54) **Vorrichtung und Verfahren zur Navigation**
Naviation device and method
Dispositif et procédé de navigation

(43) Veröffentlichungstag der Anmeldung: 29.05.2002
(73) Patentinhaber: BrainLAB AG, 85551 Kirchheim/Heimstetten (DE)
(72) Erfinder: Schmidt, Robert, 85586 Poing (DE); Dombay, Akos, 85635 Höhenkirchen (DE); Hartlep, Andreas, Dr., 80803 München (DE)
(74) Vertreter: Schwabe - Sandmair - Marx

(56) Entgegenhaltungen:
- DE-A- 3 931 531
- US-A- 5 588 430
- US-A- 5 891 034
- US-A- 5 999 840
- US-A- 6 122 541

## Beschreibung

Die vorliegende Erfindung bezieht sich auf ein Verfahren und eine Vorrichtung zur Navigation, insbesondere bei intraoperativer Datenerfassung. Allgemein bezieht sich die Erfindung auf ein Verfahren und eine Vorrichtung zum Lokalisieren eines bestimmten zum Beispiel in einem äußeren Körper liegenden Objektes, wie zum Beispiel eines Tumors, in einem definierten Koordinatensystem, so dass kontinuierlich und dynamisch, also zum Beispiel bei einer Bewegung einer Person, in welcher ein Tumor lokalisiert wurde, jederzeit genau ermittelt werden kann, wie die räumliche Lage des Tumors oder eines anderen interessierenden Bereiches ist.

Aus der DE 198 05 112 A1 ist ein Verfahren und eine Vorrichtung zum Kalibrieren eines Navigationssystems bezüglich Bilddaten eines Magnetresonanzgerätes zum Positionieren eines Patienten in einem Magnetresonanzgerät bekannt. Hierzu werden Positionen von mindestens drei in einem Abbildungsvolumen eines Magnetresonanzgerätes angeordneten Markern mit einem Navigationssystem in einem ersten Koordinatensystem und mittels Magnetresonanz in einem zweiten Koordinatensystem ermittelt, wobei aus den Positionen der Marker in beiden Koordinatensystemen Lage und Orientierung der beiden Koordinatensysteme zueinander bestimmt werden, so dass anhand einer so ermittelten Koordinatentransformationsmatrix Koordinatendaten aus dem ersten Koordinatensystem in Koordinatendaten des zweiten Koordinatensystems transformiert werden können. Dieses Verfahren ermöglicht es einen Patienten in einem Magnetresonanzgerät möglichst genau zu positionieren, so dass ein bestimmter Bereich des Patienten, in welchem zum Beispiel ein Tumor vermutet wird, mit einem Zeigeinstrument markiert werden kann, um in einer durch das Zeigeinstrument angegebenen Ebene ein Schnittbild zu erstellen. Wird der Patient nach erfolgter Aufnahme wieder aus dem Magnetresonanzgerät herausgenommen, sind lediglich die Daten bestimmter Schnittbilder bekannt, jedoch kann keine Lokalisierung eines interessierenden Objektes im Raum erfolgen, d. h., dass nach dem Herausnehmen einer Person aus dem Magnetresonanzgerät die momentane exakte Lage im Raum von interessierenden Objekten in einer Person nicht unmittelbar und präzise bestimmt werden kann.

Aus dem Artikel "Motion Compensation by Gradient Adjustment" von H. Eviatar und anderen, Institute for Biodiagnostics, National Research Council Canada, Winnipeg, Manitoba, Canada; ISMRM Procedures 1997, ist ein Verfahren bekannt um mittels Kernspinresonanz über einen bestimmten Zeitraum aufgenommene Bilder miteinander zu vergleichen. Hierzu werden Bezugselemente fest an einem Kopf befestigt, welche einen Aufnahmebereich bestimmen, so dass in Relation zu diesen Bezugselementen unabhängig von einer möglicherweise anderen Positionierung eines zu untersuchenden Kopfes relativ zu einem Magnet ermittelt werden kann, ob sich bestimmte Bereiche zum Beispiel des Hirns durch Relativbewegungen verschoben haben. Auch nach diesem Verfahren ist es nicht möglich kontinuierlich die Position eines interessierenden Bereiches unmittelbar zu bestimmen, wenn eine zu untersuchende Person wieder aus dem Kernspinresonanzgerät herausgenommen wurde.

Die US 6,122,541 offenbart ein Kopfband zur stereotaktischen Registrierung, wobei zwei verschiedene Arten von Markern an dem Kopfband vorgesehen sind, nämlich Referenzmarker, welche zum Beispiel bei einer MR-Aufnahme sichtbar sind, und zusätzliche Referenzmarker, welche optisch sichtbar sind, wobei nach einem mit einem chirurgischen Instrument durchgeführten Registrierungsverfahren eine Koordinaten-Transformation durchgeführt wird, um einen optisch geführten chirurgischen Eingriff durchführen zu können.

Aus der US 5,891,034 ist ein System zum Ermitteln der Position einer chirurgischen Sonde innerhalb eines Kopfes auf einem Bild des Kopfes bekannt, wobei mit einem optischen Scanner die Position der Stirn des Kopfes erfasst wird.

Die US 5,588,430 beschreibt die Verwendung einer Bissplatte mit daran angebrachten Leuchtdioden, um eine Positionsbestimmung durchführen zu können.

Es ist die Aufgabe der vorliegenden Erfindung ein Verfahren und eine Vorrichtung zum Bestimmen der Position eines Objektes, wie zum Beispiel eines Tumors im Hirngewebe, vorzuschlagen, welche eine kontinuierliche präzise Bestimmung der relativen Lage des interessierenden Objektes in einem Bezugskoordinatensystem ermöglichen. Insbesondere sollen ein Verfahren und eine Vorrichtung vorgeschlagen werden, mit welchen intraoperative Aufnahmen eines Körpers, der das Objekt enthält, durchgeführt werden können, wobei auch nach Entnahme des Körpers, also zum Beispiel einer zu untersuchenden Person aus beispielsweise einem Kernspinresonanz- oder Computertomographie-Gerät die exakte Position des Objekts bzw. von interessierenden bzw. aufgenommenen Bereichen in Bezug auf ein Koordinatensystem kontinuierlich bestimmt werden kann, wodurch es möglich wird eine präzise Navigation zur Unterstützung bei der Untersuchung oder Behandlung einer Person zur Verfügung zu stellen.

Diese Aufgabe wird durch ein Verfahren und eine Vorrichtung mit den Merkmalen der unabhängigen Ansprüche gelöst. Vorteilhafte Ausführungsformen ergeben sich aus den Unteransprüchen.

Bei dem erfindungsgemäßen Verfahren zum Bestimmen der Position eines Objektes in einem Navigations-Koordinatensystem, wobei allgemein unter einem Objekt auch ein oder mehrere geometrische Punkte angesehen werden können, werden ein oder mehrere Referenzpunkte, welche auch als Marker bezeichnet werden und bezüglich einer verwendeten Signalerfassungsvorrichtung ein spezifisches Ansprech- bzw. Reflexionsverhalten aufweisen, in einem definierten Position- bzw. Lageverhältnis mit dem Objekt verbunden. Dabei ist es nicht erforderlich, dass die Referenzpunkte bzw. Marker unmittelbar an dem Objekt angebracht sind, was nicht möglich ist, wenn das Objekt von einem äußeren Körper umgeben wird. Allgemein reicht das feste Anbringen bzw. Fixieren von Markern in einem möglichst stabilen, d. h. durch äußere Krafteinwirkung nicht leicht verschiebbaren Lageverhältnis zum Beispiel an dem äußeren Körper aus. Beispielsweise können ein oder mehrere Marker an einem zu untersuchenden Kopfüber eine spezielle fest an dem Kopf angebrachte Klammer vorgesehen sein. Solche Klammern sind weithin bekannt und werden auch als Skull-Clamp oder Mayfield™-Clamp bezeichnet. Die Marker können bei einem solchen Anwendungsfall entweder in die Skull-Clamp integriert sein oder an einer definierten Position in einem definierten Lageverhältnis anbringbar, zum Beispiel aufsteckbar sein. Diese fest mit dem Objekt verbundenen bzw. in einem festen definierten Lageverhältnis dazu stehenden Marker können zur Bestimmung der Position zum Beispiel des äußeren Körpers in einem Erfassungs-Koordinatensystem verwendet werden, wobei die Position bzw. Veränderungen der Lage des interessierenden im äußeren Körper liegenden Objektes, also zum Beispiel eine nach Öffnung einer Schädeldecke durch veränderte Druckverhältnisse verursachte Verschiebung eines Hirnbereiches, auch als "Brainshift" bezeichnet, ebenfalls im Erfassungs-Koordinatensystem, erfasst werden. Hierzu wird zunächst die Position des Objektes in Bezug auf das Erfassungs-Koordinatensystem, also zum Beispiel in Bezug auf ein Koordinatensystem eines Kernspinresonanzgerätes bzw. des Erfassungsbereiches eines Bildaufnahmegerätes, bestimmt bzw. aufgenommen, wobei die Position der Referenzpunkte bzw. Marker ebenfalls in Bezug auf dieses Erfassungs-Koordinatensystem bestimmt wird. Die Erfassung der Position der Marker kann vor, nach oder gleichzeitig mit der Positionserfassung des Objektes erfolgen. Somit kann ein relatives Lageverhältnis zwischen dem erfassten Objekt und den erfassten Referenzpunkten bzw. Markem ermittelt werden, so dass die Lage des Objektes eindeutig definiert ist, wenn die Koordinaten der Referenzpunkte bzw. Marker bekannt sind. Anschließend wird die Position der Marker in Bezug auf ein Navigations-Koordinatensystem bestimmt, so dass eine genaue Lokalisierung der Marker im Raum möglich ist, d. h., es werden die Koordinaten des bzw. der Marker in Bezug auf ein zur Navigation dienendes Koordinatensystem bestimmt. Da zuvor das relative Lage- bzw. Positionsverhältnis zwischen Objekt und Markern zum Beispiel in einem Kernspintomographen ermittelt wurde, kann unter Verwendung der Navigations-Koordinaten der Marker ermittelt werden, wie die absolute Lage des Objektes im Raum ist. Diese Lagebestimmung des Objektes kann kontinuierlich erfolgen, d. h. zum Beispiel, dass auch nach der Entnahme einer Person aus einem Kernspintomographen kontinuierlich ermittelt werden kann, wo sich in Bezug auf ein Navigations-Koordinatensystem das interessierende Objekt, zum Beispiel ein Tumor oder ein anderer Gewebebereich, im Raum befindet, da das Navigations-Koordinatensystem in einem festen und bekannten Verhältnis zum Messobjekt steht. Dies kann für eine Untersuchung oder Behandlung vorteilhaft sein, da zum Beispiel bei gleichzeitiger Erfassung der Lage eines Behandlungsinstrumentes im Raum dieses Behandlungsinstrument in eine gewünschte Position relativ zu dem Objekt gebracht werden kann. Die Position des interessierenden Objektes wird erfindungsgemäß also nicht "verloren" wie dies bei den oben beschriebenen Verfahren nach dem Stand der Technik der Fall ist, sondern kann zum Beispiel kontinuierlich bestimmt werden.

Im Sinne der Erfindung ist es jedoch nicht erforderlich die gleichen Referenzpunkte bzw. Marker zu verwenden, um in einem ersten Schritt das Lageverhältnis zwischen Markern und Objekt und in einem zweiten Schritt aus der erfassten Position von Markern die Position des Objektes zu ermitteln. Es können auch verschiedene Marker bzw. Referenzpunkte für zum Beispiel eine Kernspinresonanz-Aufnahme und eine IR-Navigation verwendet werden, wenn diese zum Beispiel in einem definierten bekannten Lageverhältnis zueinander stehen. Natürlich ist es auch möglich die gleichen Marker zu verwenden bzw. Marker mit bestimmten für das jeweilige Erfassungsverfahren günstigen Eigenschaften miteinander zu kombinieren bzw. ineinander zu integrieren.

Erfindungsgemäß ist es demzufolge möglich kontinuierlich die exakte Position eines interessierenden Objektes in Bezug auf ein Navigations-Koordinatensystem zu erfassen, so dass auch bei einer Bewegung des Objektes bzw. eines äußeren Körpers, also zum Beispiel einer Person, die exakte Position des Objektes bestimmt werden kann, d. h., dass dieses Objekt getrackt werden kann. Es ist jedoch nicht erforderlich eine kontinuierliche Positionsbestimmung vorzunehmen, so dass auch intermittierende bzw. zeitdiskret arbeitende Verfahren verwendet werden können, um die Position des Objektes zu bestimmten Zeitpunkten zu bestimmen, falls eine kontinuierliche Erfassung nicht erforderlich ist.

Vorteilhaft wird bei der Erfassung des Objektes in einer dafür geeigneten Vorrichtung zunächst eine anatomische Aufnahme, zum Beispiel eine Groberfassung bzw. ein Überblickscan durchgeführt, d. h., dass zum Beispiel eine Auflösung eines größeren erfassten Bereiches (FOV "Field of view") unterhalb der gewünschten Auflösung zum Erhalten eines gewünschten Bildes oder einer optimalen Bildsequenz liegen kann. Mit einem solchen Überblickscan bzw. Localizer ist es zum Beispiel möglich anhand einer ersten aufgenommenen Bildfolge eines größeren Bereiches zum Beispiel in relativ grober Auflösung die Fokussierung der Erfassungsvorrichtung auf einen gewünschten kleineren Bereich von Hand oder automatisch einzustellen, so dass Bilder aus diesem gewünschten Bereich, nach erfolgter Fokussierung mit einer zweiten Bildgebungssequenz oder Spektroskopie zum Beispiel in höherer Auflösung oder anderer funktioneller Aussage aufgenommen werden können. Mittels Navigations-Koordinatensystem und der Position der Marker in Bezug auf das Erfassungs-Koordinatensystem kann somit zum Beispiel auch von außerhalb des Magneten zum Beispiel ein zweites detaillierteres Scan-Verfahren eines interessierenden Bereiches durch automatisches Fokussieren auf einen durch die Marker bestimmten Bereich durchgeführt werden. Somit ist es auch möglich, gegebenenfalls unter Verwendung von bildauswertenden oder spektroskopischen Verfahren, eine automatische Fokussierung auf einen interessierenden Bereich für eine beispielsweise detailliertere zweite Aufnahme durchzuführen.

In einer vorteilhaften Ausführungsform kann im Navigations-Koordinatensystem ein Bereich in Bezug auf das Objekt festgelegt werden, zum Beispiel durch eine behandelnde Person, welche mittels eines Zeigeinstrumentes im Navigations-Koordinatensystem markiert, innerhalb dessen Daten, insbesondere Bilddaten, im Erfassungs-Koordinatensystem erfasst werden. Ist zum Beispiel ein Patient aus einem Kernspintomographen oder einer anderen geeigneten bildgebenden Vorrichtung herausgenommen worden und wird zum Beispiel die Lage eines im Patienten lokalisierten Tumors im Navigations-Koordinatensystem bestimmt, so kann zum Beispiels mittels eines Zeigeinstrumentes, dessen Position ebenfalls im Navigations-Koordinatensystem bestimmt wird, ein bestimmter Bereich in der Person gekennzeichnet werden, welcher beispielsweise den Tumor enthält, so dass bei einem erneuten Einbringen des Patienten in zum Beispiel einen Kernspintomographen automatisch auf diesen im Navigations-Koordinatensystem festgelegten Bereich fokussiert werden kann. Somit kann anhand der Position der fest mit dem interessierenden Objekt verbundenen Marker und dem im Navigations-Koordinatensystem definierten Bereich eine Fokussierung oder gewünschte Einstellung auf einen interessierenden Bereich vorgenommen werden, so dass ein bildgebendes Verfahren Daten bezüglich des so bestimmten Bereiches liefern kann.

Bevorzugt wird zur Ermittlung der räumlichen Lage bzw. Position oder Orientierung des Objektes in einem Erfassungs-Koordinatensystem bzw. relativ zu Markern ein bildgebendes Verfahren, insbesondere ein Kernspinresonanz-, Ultraschall- oder ein Computertomographie-Verfahren verwendet. Jedoch ist es allgemein möglich jedes andere Verfahren zu verwenden, welches die Bestimmung der Position eines Objektes, welches gegebenenfalls in einem größeren äußeren umgebenden Objekt angeordnet ist, zusammen mit der Bestimmung der Position eines oder mehrerer Referenzpunkte bzw. Marker zu ermöglichen. Geeignete Verfahren, um die Position eines oder mehrerer Marker in einem Navigations-Koordinatensystem zu bestimmen, können zum Beispiel Infrarotlicht verwenden, so dass zum Beispiel IR-reflektierende Marker von IR-Kameras erfasst werden. Es ist jedoch auch möglich auf Ultraschall oder Funk basierende bzw. elektromagnetische Verfahren zu verwenden. Allgemein kann jedes Verfahren verwendet werden, welches es ermöglicht die exakte Position eines oder mehrerer Punkte in Bezug auf ein festes Koordinatensystem zu ermitteln.

Es ist von Vorteil ein Koordinaten-Transformationsverfahren zu verwenden, um in dem Erfassungs-Koordinatensystem erhaltene Positionsdaten des Objektes oder der Marker in das Navigations-Koordinatensystem umzurechnen. Diesbezüglich wird auf die Lehre der DE 198 05 112 A1 verwiesen, deren Offenbarung bezüglich der dort nur für die Positionierung beim Kemspinresonanz-Verfahren vorgenommenen Bestimmung einer Transformationsmatrix zur Koordinatentransformation für die erfindungsgemäße allgemeinen Verwendung in diese Beschreibung aufgenommen wird.

Vorteilhaft wird durch mindestens eine Erfassungsvorrichtung zur Bestimmung der Lage im Erfassungs- oder Navigations-Koordinatensystem auch noch die Position mindestens eines weiteren Instrumentes erfasst, wie zum Beispiel die Position eines Skalpells anhand von daran angebrachten Markern, so dass das Lageverhältnis zwischen Objekt und Instrument ermittelt und ggf. visualisiert werden kann, um zum Beispiel automatisch eine Positionierung bzw. Führung eines Instrumentes durchzuführen oder um durch geeignete bildliche Darstellung einer Person Informationen bezüglich des relativen Lageverhältnisses von Instrument und Objekt zur Verfügung zu stellen, um das Instrument möglichst präzise, d. h. genau positioniert einsetzen zu können.

Die erfindungsgemäße Vorrichtung zum Bestimmen der Position eines Objektes oder Punktes in einem Navigations-Koordinatensystem weist eine Vorrichtung zum ortsfesten, d. h. in einem festen Lageverhältnis stehenden Befestigen von Referenzpunkten bzw. Markern an dem Objekt oder an einem das Objekt umgebenden Körper auf. Weiterhin ist eine Vorrichtung zur Erfassung der Position des Objektes zusammen mit der Position des bzw. der Referenzpunkte bzw. Marker in einem Erfassungs-Koordinatensystem vorgesehen. Erfindungsgemäß ist eine Vorrichtung zur Erfassung der Position des bzw. der Marker oder Referenzpunkte in einem Navigations-Koordinatensystem vorgesehen, wobei die Erfassungsvorrichtungen zur Bestimmung der Position im Erfassungs-Koordinatensystem und zur Bestimmung der Position im Navigations-Koordinatensystem auf verschiedenen physikalischen Prinzipien basieren können. Mit einer Datenverarbeitungsvorrichtung kann die Position des Objektes im Navigations-Koordinatensystem aus der erfassten Position des bzw. der Referenzpunkte im Navigations-Koordinatensystem und dem relativen Lageverhältnis zwischen Objekt und Marker, welches im Erfassungs-Koordinatensystem bestimmt wurde, ermittelt werden. Die erfindungsgemäße Vorrichtung arbeitet im Wesentlichen nach oben beschriebenem Verfahren, so dass diesbezüglich hierauf verwiesen wird.

Vorteilhaft ist die Erfassungsvorrichtung zur Bestimmung der Position eines Referenzpunktes bzw. Markers im Navigations-Koordinatensystem außerhalb der Erfassungsvorrichtung zur Positionsbestimmung des Objektes und der Marker im Erfassungs-Koordinatensystem angebracht. Es sind also zum Beispiel Infrarotkameras außerhalb eines Kernspintomographen angeordnet, um nach Durchführung einer Kernspintomographie eine Positionsbestimmung eines interessierenden Objektes im Raum außerhalb des Kernspintomographen durchführen zu können. Jedoch ist es auch denkbar die zur Erfassung der Position im Navigations-Koordinatensystem dienende Erfassungsvorrichtung integral mit der zur Erfassung der Position im Erfassungs-Koordinatensystem dienenden Vorrichtung auszubilden, d. h. zum Beispiel eine oder mehrere Infrarot-Kameras in einen Kernspin-Tomographen zu integrieren.

Bevorzugt wird als Vorrichtung zum ortsfesten Anbringen von Referenzpunkten bzw. Markern an einem interessierenden Objekt eine Skull-Clamp verwendet, wobei die Marker entweder fest an der Skull-Clamp angeordnet bzw. in diese integriert sein können oder auf diese aufsteckbar sein können. Da es erfindungsgemäß möglich ist auf zwei unterschiedlichen physikalischen Prinzipien basierende Erfassungsvorrichtungen für die jeweiligen Koordinatensysteme zu verwenden, können die einzelnen Marker auch getrennt voneinander vorgesehen sein, d. h. dass zum Beispiel ein erster Marker-Typ in die Skull-Clamp integriert ist, wobei ein zweiter Marker-Typ aufsteckbar ist. Jedoch können auch alle Marker integriert oder aufsteckbar ausgebildet sein. Dabei ist es prinzipiell auch möglich Marker für verschiede Erfassungssysteme ineinander zu integrieren, d. h. zum Beispiel geeignete Spulenelemente oder Beschichtungen um durch Magnetresonanz nachweisbare Substanzen herum anzuordnen. Als weitere Ausgestaltung der Erfindung ist es auch möglich IR-reflektierende Elemente an durch magnetresonanznachweisbaren Substanzen vorzusehen.

Obwohl die Erfindung oben beispielhaft anhand einer Skull-Clamp beschrieben wurde, können auch andere Vorrichtungen an einem interessierenden bzw. zu untersuchenden Objekt vorgesehen sein, welche mit geeigneten Markern versehen sind, wie zum Beispiel Schienen zur Untersuchung einer Knochen- oder Knorpelstruktur oder ähnliche Geräte, sofern diese ortsfest angebracht, d. h. in ein definiertes Lageverhältnis zu dem interessierenden Objekt gebracht werden können.

Bevorzugt werden mindestens zwei Marker für jede Erfassungsvorrichtung vorgesehen, wobei es von Vorteil ist drei oder sogar vier oder mehr Marker zu verwenden, welche vorteilhaft nicht in einer Ebene liegen, um eine eindeutige räumliche Zuordnung zu ermöglichen. Die Marker können dabei alle gleich ausgebildet sein, d. h. gleiche Form, Reflexions- oder Absorptionseigenschaften aufweisen, je nach Art der verwendeten Erfassungstechnik. Weiterhin ist es auch möglich verschieden ausgestaltete Marker zu verwenden, so dass beispielsweise anhand der Größe, des Reflexionsgrades, der Geometrie oder anderer unterscheidender Merkmale erkannt werden kann, welcher spezifische Marker aus einer Gruppe von mehreren Markern in einem Erfassungsvorgang aufgenommen wurde.

Bevorzugt ist eine Anzeigevorrichtung vorgesehen, welche die Position des Objektes im Raum bzw. die Lage im Navigations-Koordinatensystem, gegebenenfalls in Relation zu einem oder mehreren Instrumenten, deren Position ebenfalls gegebenenfalls mittels geeigneter Marker erfasst wurde, darstellen zu können. Es kann zum Beispiel durch die Darstellung des relativen Lageverhältnisses zwischen Objekt und einem Instrument in einer oder mehrerer Betrachtungsebenen und/oder einer dreidimensionalen Darstellung die Einsatzmöglichkeit bzw. Präzision des Einsatzes eines solchen Instruments verbessert werden.

Bevorzugt wird als Erfassungsvorrichtung zum Bestimmen der Position des Objektes und geeigneter Marker in einem Erfassungs-Koordinatensystem eine Vorrichtung zur Durchführung einer Computertomographie oder eines Kernspinresonanz-Verfahrens verwendet. Allgemein können jedoch, wie oben ausgeführt, andere Vorrichtungen verwendet werden, welche die Positionsbestimmung von Objekten, welche gegebenenfalls nicht frei zugänglich sind, d. h. von Materie oder Gewebe umgeben sind, ermöglichen.

Die Vorrichtung zur Erfassung der Position eines Referenzpunktes oder Markers im Navigations-Koordinatensystem kann eine oder mehrere Infrarot-Kameras gegebenenfalls zusammen mit geeigneten IR-Lampen, Sender- und Empfänger-Anordnungen zur Positionsbestimmung über Funk, Lautsprecher und/oder Mikrofone zur Durchführung von auf Schall basierenden Positionsbestimmungsverfahren oder eine andere geeignete Vorrichtung sein, welche die möglichst genaue Bestimmung der Position eines oder mehrerer Punkte in einem Koordinatensystem ermöglichen.

Vorteilhaft ist mindestens eine der Erfassungsvorrichtungen beweglich ausgestaltet, d. h., dass zum Beispiel der Kernspintomograph über einen Patienten verschoben werden kann, um intraoperativ Aufnahmen gegebenenfalls nach Durchführung eines Scout-Views tätigen zu können. Ebenso ist es möglich zum Beispiel auch bewegliche Infrarot-Kameras zur Bestimmung der Position im Navigations-Koordinatensystem vorzusehen.

Die Erfindung wird nachfolgend für eine spezielle Ausführungsform anhand von Figuren beschrieben werden. Es zeigen:
- Figur 1: eine schematische Darstellung einer erfindungsgemäßen Vorrichtung; und
- Figur 2: ein allgemeines Ablaufdiagramm des erfindungsgemäßen Verfahrens.

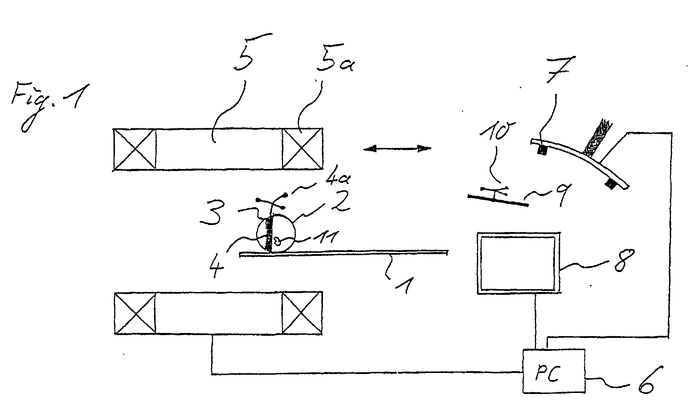

Figur 1 zeigt schematisch eine Auflage 1, auf welcher ein zu untersuchendes Objekt, also beispielsweise ein Kopf eines Patienten liegt, an welchem eine Skull-Clamp 3 mit einer Mehrzahl von integrierten Markern 4 und aufsteckbaren Markern 4a befestigt ist, die in ihrem Inneren eine bei einem Kernspinresonanz-Verfahren gut sichtbare Substanz aufweisen, wobei die Marker 4a mit einer für Infrarot-Kameras gut erfassbaren Beschichtung versehen sind und in festem bzw. wiederherstellbarem Bezug zu den integrierten Markern 4 stehen. Die Auflage 1 kann in ein Kernspinresonanz-Gerät 5 mit geeigneten Wicklungen 5a eingeschoben werden oder die Kernspinresonanz-Vorrichtung kann über die Auflage 1 geschoben werden, um präoperativ oder intraoperativ Aufnahmen durchzuführen. Die dabei erfassten Positionsdaten zum Beispiel eines innerhalb des Kopfes 2 liegenden Tumors 11 werden zusammen mit den Positionsdaten der Marker 4 an einen Rechner 6 übermittelt, welcher aus den Positionen der Marker 4 und der Position des Objektes 11 in einem Erfassungs-Koordinatensystem das relative Lageverhältnis bestimmen kann.

Wenn das Kernspinresonanz-Verfahren beendet ist wird die Position der Marker 4a in einem Navigations-Koordinatensystem durch Infrarot-Kameras 7 erfasst, wobei die Positionsdaten ebenfalls an einen Rechner 6 weitergeleitet werden. Der Rechner 6 kann mittels der relativen Positionsdaten, welche von dem Kernspintomographen 5 erhalten wurden, kontinuierlich die Position der Marker 4a, und damit der Skull-Clamp 3 bzw. des Objektes unter Verwendung der von den Infrarot-Kameras 7 kommenden Positionsdaten ermitteln, um so die Position des in dem Körper 2 befindlichen Objektes 11 im Raum in Bezug auf ein Navigations-Koordinatensystem zu bestimmen. Die Lage des Körpers 2 bzw. des in dem Körper 2 angeordneten Objektes 11, wie zum Beispiel eines Tumors, in Bezug auf ein Navigations-Koordinatensystem kann an einem Bildschirm 8 dreidimensional und/oder in verschiedenen 2D-Betrachtungsebenen dargestellt werden. Ergänzend kann die Position eines Instruments 9 mit daran angebrachten Markern 10, welche ebenfalls von den Infrarot-Kameras 7 erfasst werden, auf dem Bildschirm 8 dargestellt werden, um so eine möglichst genaue Positionierung eines Instruments 9 in Bezug auf das Objekt 11, also zum Beispiel die möglichst exakte Führung eines Skalpells zu einem Hirntumor, zu ermöglichen.

Treten aufgrund von Eingriffen bzw. Veränderungen an dem Körper 2 möglicherweise Veränderungen der Position des in dem Körper 2 befindlichen Objektes 11 auf, so kann intraoperativ eine weitere Datenerfassung zur Bestimmung der momentanen relativen Position mittels des Kernspintomographen 5 erfolgen, wodurch die Positionsänderungen des Objektes 11 kompensiert werden können.

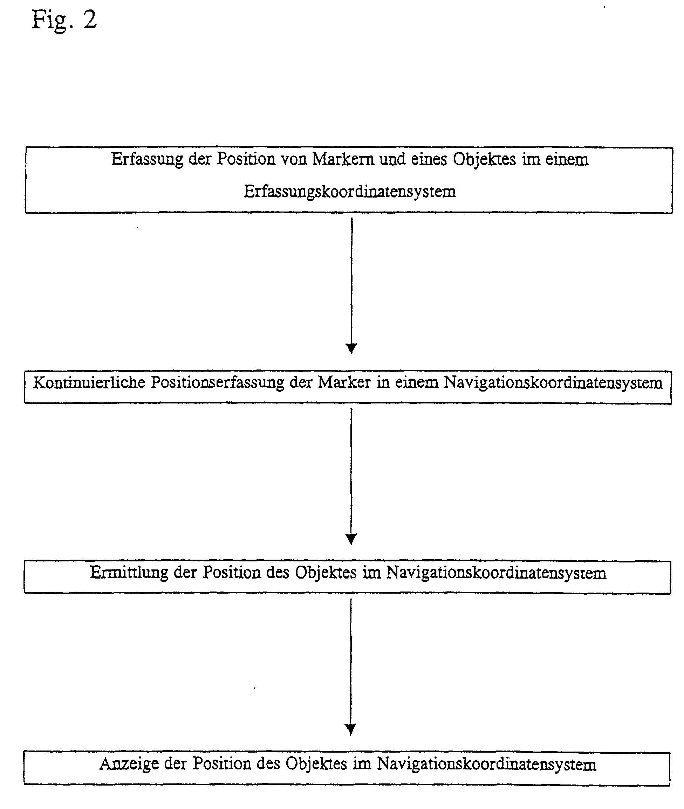

Figur 2 zeigt allgemein das Prinzip des erfindungsgemäßen Verfahrens, wobei in einem ersten Schritt präoperativ oder intraoperativ die Position eines Objektes zusammen mit der Position eines mehrerer Marker in einem Erfassungs-Koordinatensystem innerhalb zum Beispiel des in Figur 1 gezeigten Kernspintomographen 5 erfolgt. Nach Beendigung der Datenerfassung zum Beispiel in dem Kernspintomographen wird eine kontinuierliche Positionserfassung der Marker 4a mittels der Infrarot-Kameras 7 in einem Navigations-Koordinatensystem durchgeführt, wobei aus diesen Daten unter Berücksichtigung des zuvor im Erfassungs-Koordinatensystem bestimmten relativen Lageverhältnisses zwischen Objekt und Markern die Position des Objektes im Navigations-Koordinatensystem kontinuierlich ermittelt werden kann, so dass es möglich ist kontinuierlich die momentane Position des Objektes im Navigations-Koordinatensystem anzuzeigen.

Besteht aufgrund von Eingriffen oder Veränderungen an dem Körper 2 die Möglichkeit einer Veränderung des Lageverhältnisses zwischen Objekt und Markern, so kann intraoperativ eine Kernspinresonanz-Aufnahme durchgeführt werden, um das neue exakte relative Lageverhältnis zwischen Objekt und Markern 4 zu erhalten, so dass hierdurch die Genauigkeit der Positionsbestimmung des Objektes verbessert werden kann.

## Patentansprüche

1. Verfahren zum Bestimmen der Position mindestens eines Objektes in einem Navigations-Koordinatensystem, wobei
a) mindestens ein Referenzpunkt bzw. Marker (4) in einem definierten Lageverhältnis mit dem Objekt (11) verbunden wird;
b) die Position des Objektes (11) in Bezug auf ein Erfassungs-Koordinatensystem erfasst wird;
c) die Position des mindestens einen Referenzpunktes (4) in einem Navigations-Koordinatensystem erfasst wird;
**dadurch gekennzeichnet, dass**
d) die Position des mindestens einen Referenzpunktes (4) in Bezug auf das Erfassungs-Koordinatensystem erfasst wird; und
e) die Position des Objektes (11) im Navigations-Koordinatensystem aus der Position des mindestens einen Referenzpunktes (4) im Navigations-Koordinatensystem und der aus den Schritten b) und d) ermittelten relativen Lage des Objekts (11) zu dem mindestens einen Referenzpunkt (4) bestimmt wird.

2. Verfahren nach Anspruch 1, wobei die Positionsbestimmung des Objektes (11) im Navigations-Koordinatensystem kontinuierlich erfolgt.

3. Verfahren nach Anspruch 1 oder 2, wobei vor der Erfassung der Position des Objektes (11) und/oder des mindestens einen Referenzpunktes (4) im Erfassungs-Koordinatensystem mit der gewünschten Bildauflösung eine Erfassung anatomischer Bilddaten durchgeführt wird.

4. Verfahren nach einem der vorhergehenden Ansprüche, wobei im Navigations-Koordinatensystem ein Bereich in Bezug auf das Objekt (11) festgelegt wird, innerhalb dessen Daten, insbesondere Bilddaten, im Erfassungs-Koordinatensystem erfasst werden.

5. Verfahren nach einem der vorhergehenden Ansprüche, wobei die Positionserfassung im Erfassungs-Koordinatensystem durch bildgebende Verfahren, insbesondere Kernspinresonanz-, Ultraschall- oder Computertomographie-Verfahren erfolgt.

6. Verfahren nach einem der vorhergehenden Ansprüche, wobei die Positionserfassung im Navigations-Koordinatensystem durch Infrarot-, Schall-, Ultraschall- oder elektromagnetische, insbesondere Funk-Verfahren erfolgt.

7. Verfahren nach einem der vorhergehenden Ansprüche, wobei eine Koordinatentransformationsmatrix zur Umrechnung von Erfassungskoordinaten in Navigationskoordinaten ermittelt wird.

8. Verfahren nach einem der vorhergehenden Ansprüche, wobei die Position weiterer Referenzpunkte bzw. Marker (10) und/oder mindestens eines Instrumentes (9) im Erfassungs- und/oder Navigations-Koordinatensystem erfasst wird.

9. Vorrichtung zur Bestimmung der Position mindestens eines Objektes in einem Navigations-Koordinatensystem mit:
a) einer Befestigungsvorrichtung (3), welche in einem definierten Lageverhältnis am Objekt (11) angebracht werden kann;
b) einer Vorrichtung (5, 5a) zur Erfassung der relativen Position des Objektes (11) und mindestens eines in einem definierten Lageverhältnis zum Objekt (11) an der Befestigungsvorrichtung (3) befestigten Markers (4) in einem Erfassungs-Koordinatensystem;
c) einer Vorrichtung (7) zur Erfassung der Position des mindestens einen Markers (4) in einem Navigations-Koordinatensystem; und
d) einer Datenverarbeitungsvorrichtung (6) zur Bestimmung der Position des Objektes (11) im Navigations-Koordinatensystem aus der erfassten Position des Objektes (11) relativ zu dem mindestens einen Marker (4) im Erfassungs-Koordinatensystem und aus der erfassten Position des mindestens einen Markers (4) im Navigations-Koordinatensystem.

10. Vorrichtung nach Anspruch 9, wobei die Erfassungsvorrichtung (7) zur Bestimmung einer Position in einem Navigations-Koordinatensystem außerhalb der Erfassungsvorrichtung (5) zur Bestimmung einer Position in einem Erfassungs-Koordinatensystem angebracht ist.

11. Vorrichtung nach Anspruch 9 oder 10, wobei der mindestens eine Marker (4) fest an der an dem Objekt (2) angebrachten Befestigungsvorrichtung (3) angeordnet oder in diese integriert ist.

12. Vorrichtung nach einem der Ansprüche 9 bis 11, wobei mindestens ein Marker (4) auf die ortsfest angebrachte Befestigungsvorrichtung (3) aufgesteckt werden kann.

13. Vorrichtung nach einem der Ansprüche 9 bis 12, wobei der mindestens eine Marker (4) eine bei einem Magnetresonanz-Verfahren und/oder bei einem Computertomographie-Verfahren detektierbare Substanz enthält und/oder eine in einem bestimmten Spektralbereich gut reflektierende bzw. sichtbare Beschichtung aufweist.

14. Vorrichtung nach einem der Ansprüche 9 bis 13, wobei mindestens zwei Marker (4), insbesondere drei Marker (4), welche nicht auf einer Geraden liegen, oder vier Marker (4), welche nicht in einer Ebene liegen, oder mehr als vier Marker (4) an der Befestigungsvorrichtung (3) vorgesehen sind.

15. Vorrichtung nach Anspruch 14, wobei alle Marker (4) gleich ausgebildet sind oder mindestens ein Marker (4) aus einer Mehrzahl von Markern (4) bezüglich der Geometrie, des Reflexionsverhaltens, des Absorptionsverhaltens oder anderer Unterscheidungsmerkmale anders ausgebildet ist als die anderen Marker (4).

16. Vorrichtung nach einem der Ansprüche 9 bis 15, wobei eine Anzeigevorrichtung (8) zur Darstellung der ermittelten Positionen des Objekts (11) im Navigations-Koordinatensystem vorgesehen ist.

17. Vorrichtung nach einem der Ansprüche 9 bis 16, wobei zur Erfassung der Position im Erfassungs-Koordinatensystem und/oder im Navigations-Koordinatensystem eine Vorrichtung (5) zur Durchführung eines Kernspinresonanz-Verfahrens, eine Vorrichtung zur Durchführung eines Computertomographie-Verfahrens und/oder eine Vorrichtung (7) zur Erfassung von Licht, insbesondere Infrarot-Signalen, Funksignalen, oder Schallsignalen vorgesehen ist.

18. Vorrichtung nach einem der Ansprüche 9 bis 17, wobei mindestens eine der Erfassungsvorrichtungen (5, 7) zum Bestimmen der Position im Erfassungs- und/oder Navigations-Koordinatensystem beweglich ausgestaltet ist.

## Claims

1. A method for determining the position of at least one object in a navigation system of co-ordinates, wherein:
a) at least one reference point or marker (4) is connected to said object (11) in a defined positional relationship;
b) said position of said object (11) is detected in relation to a detection system of co-ordinates;
c) said position of said at least one reference point (4) is detected in a navigation system of co-ordinates;
**characterised in that**
d) said position of said at least one reference point (4) is detected in relation to said detection system of co-ordinates; and
e) said position of said object (11) in said navigation system of co-ordinates is determined from said position of said at least one reference point (4) in said navigation system of co-ordinates and from the position of said object (11) relative to said at least one reference point (4), as determined in steps b) and d).

2. The method as set forth in claim 1, wherein said position of said object (11) is continuously determined in said navigation system of co-ordinates.

3. The method as set forth in claim 1 or 2, wherein anatomical image data are detected, before said position of said object (11) and/or of said at least one reference point (4) is detected in said detection system of co-ordinates in the desired image resolution.

4. The method as set forth in any one of the preceding claims, wherein an area is specified in said navigation system of co-ordinates in relation to said object (11), within which area data, in particular image data, are detected in said detection system of co-ordinates.

5. The method as set forth in any one of the preceding claims, wherein said position is detected in said detection system of co-ordinates by imaging methods, in particular nuclear spin resonance, ultrasound or computer tomography methods.

6. The method as set forth in any one of the preceding claims, wherein said position is detected in said navigation system of co-ordinates by infrared, sound, ultrasonic or electromagnetic, in particular radio, methods.

7. The method as set forth in any one of the preceding claims, wherein a coordinate transformation matrix is determined, for converting detection co-ordinates into navigation co-ordinates.

8. The method as set forth in any one of the preceding claims, wherein the position of further reference points and/or markers (10), and/or at least one instrument (9), is detected in said detection and/or said navigation system of co-ordinates.

9. A device for determining the position of at least one object in a navigation system of co-ordinates, comprising:
a) a fixing device (3) which may be arranged in a defined positional relationship on the object (11);
b) a device (5, 5a) for detecting the relative position of said object (11) and of at least one marker (4) fixed to said fixing device (3) in a defined positional relationship to said object (11), in a detection system of co-ordinates;
c) a device (7) for detecting the position of said at least one marker (4) in a navigation system of co-ordinates; and
d) a data processing device (6) for determining said position of said object (11) in said navigation system of co-ordinates from said detected position of said object (11) relative to said at least one marker (4) in said detection system of co-ordinates and from said recorded position of said at least one marker (4) in said navigation system of co-ordinates.

10. The device as set forth in claim 9, wherein said detection device (7) for determining a position in a navigation system of co-ordinates is arranged outside of said detection device (5) for determining a position in a detection system of co-ordinates.

11. The device as set forth in claim 9 or 10, wherein said at least one marker (4) is arranged on said fixing device (3) firmly attached to or integrated in said object (2).

12. The device as set forth in any one of claims 9 to 11, wherein at least one marker (4) can be attached to said securely attached fixing device (3).

13. The device as set forth in any one of claims 9 to 12, wherein said at least one marker (4) contains a substance which is detectable in a magnetic resonance method and/or in a computer tomography method, and/or comprises a coating which is clearly reflected or visible in a specified spectral range.

14. The device as set forth in any one of claims 9 to 13, wherein at least two markers (4), in particular three markers (4), which do not lie in a line, or four markers (4) which do not lie in a plane, or more than four markers (4), are provided on said fixing device (3).

15. The device as set forth in claim 14, wherein all markers (4) are constructed the same, or at least one marker (4) from a number of markers (4) is constructed differently to the other markers (4) with respect to geometry, reflection properties, absorption properties or other distinguishing features.

16. The device as set forth in any one of claims 9 to 15, wherein a display device (8) is provided for displaying said detected position of said object (11) in said navigation system of co-ordinates.

17. The device as set forth in any one of claims 9 to 16, wherein to detect said position in said detection system of co-ordinates and/or in said navigation system of co-ordinates, a device (5) for performing a nuclear spin resonance procedure, a device for performing a computer tomography procedure and/or a device (7) for the detecting light, in particular infrared signals, radio signals or sonic signals, is provided.

18. The device as set forth in any one of claims 9 to 17, wherein at least one of said detection devices (5, 7) is designed to be movable, for determining said position in said detection and/or navigation system of co-ordinates.

## Revendications

1. Procédé de détermination de la position d'au moins un objet dans un système de coordonnées de navigation,
a) au moins un point de référence ou marqueur (4) étant relié dans un rapport de position défini à l'objet (11);
b) la position de l'objet (11) étant acquise par rapport à un système de coordonnées d'acquisition;
c) la position du au moins un point de référence (4) étant acquise dans un système de coordonnées de navigation;
**caractérisé en ce que**
d) la position du au moins un point de référence (4) est acquise par rapport au système de coordonnées d'acquisition; et
e) la position de l'objet (11) dans le système de coordonnées de navigation est déterminée à partir de la position du au moins un point de référence (4) dans le système de coordonnées de navigation et de la position relative de l'objet (11) par rapport à au moins le point de référence (4) déterminée lors des étapes b) et d).

2. Procédé suivant la revendication 1, la détermination de position de l'objet (11) dans le système de coordonnées de navigation se faisant en continu.

3. Procédé suivant la revendication 1 ou 2, une acquisition de données d'images anatomiques étant effectuée avec la résolution d'image désirée avant l'acquisition de la position de l'objet (11) et/ou du au moins un point de référence (4) dans le système de coordonnées d'acquisition.

4. Procédé suivant l'une des revendications précédentes, une zone étant déterminée par rapport à l'objet (11) dans le système de coordonnées de navigation, à l'intérieur de laquelle des données, en particulier des données d'image, sont acquises dans le système de coordonnées d'acquisition.

5. Procédé suivant l'une des revendications précédentes, l'acquisition de la position dans le système de coordonnées d'acquisition se faisant par des procédés d'imagerie, en particulier par des procédés de résonance magnétique nucléaire, par ultrasons ou de tomographie assistée par ordinateur.

6. Procédé suivant l'une des revendications précédentes, l'acquisition de la position dans le système de coordonnées de navigation se faisant par des procédés par infrarouges, soniques, par ultrasons ou électromagnétiques, en particulier par radio.

7. Procédé suivant l'une des revendications précédentes, une matrice de transformation de coordonnées étant déterminée pour la conversion de coordonnées d'acquisition en coordonnées de navigation.

8. Procédé suivant l'une des revendications précédentes, la position d'autres points de référence ou marqueurs (10) et/ou d'au moins un instrument (9) étant acquise dans le système de coordonnées d'acquisition et/ou de navigation.

9. Dispositif de détermination de la position d'au moins un objet dans un système de coordonnées de navigation, comprenant:
a) un dispositif de fixation (3) qui peut être amené sur l'objet (11) dans un rapport de position défini;
b) un dispositif (5, 5a) d'acquisition dans un système de coordonnées d'acquisition de la position relative de l'objet (11) et d'au moins un marqueur (4) fixé au dispositif de fixation (3), se trouvant dans un rapport de position défini par rapport à l'objet (11);
c) un dispositif (7) d'acquisition de la position du au moins un marqueur (4) dans un système de coordonnées de navigation; et
d) un dispositif de traitement des données (6) pour la détermination de la position de l'objet (11) dans le système de coordonnées de navigation à partir de la position acquise de l'objet (11) relativement à au moins un marqueur (4) dans le système de coordonnées d'acquisition et de la position acquise du au moins un marqueur (4) dans le système de coordonnées de navigation.

10. Dispositif suivant la revendication 9, le dispositif d'acquisition (7) de détermination d'une position dans un système de coordonnées de navigation étant fixé en dehors du dispositif d'acquisition (5) de détermination d'une position dans un système de coordonnées d'acquisition.

11. Dispositif suivant la revendication 9 ou 10, le au moins un marqueur (4) étant disposé sur le dispositif de fixation (3) attaché de manière stationnaire à l'objet (2) ou intégré dans celui-ci.

12. Dispositif suivant l'une des revendications 9 à 11, au moins un marqueur (4) pouvant être enfiché sur le dispositif de fixation (3) attaché de manière stationnaire.

13. Dispositif suivant l'une des revendications 9 à 12, le au moins un marqueur (4) contenant une substance détectable par un procédé de résonance magnétique et/ou par un procédé de tomographie assistée par ordinateur et/ou un revêtement bien réfléchissant ou visible dans un domaine spectral déterminé.

14. Dispositif suivant l'une des revendications 9 à 13, au moins deux marqueurs (4), en particulier trois marqueurs (4), qui ne sont pas situés sur une ligne droite, ou quatre marqueurs (4), qui ne sont pas situés dans un plan, ou plus de quatre marqueurs (4) étant prévus au dispositif de fixation (3).

15. Dispositif suivant la revendication 14, tous les marqueurs (4) étant de même conception ou au moins un marqueur (4) d'une pluralité de marqueurs (4) étant différents des autres marqueurs (4) en ce qui concerne la géométrie, le comportement en réflexion, le comportement en absorption ou un autre caractère distinctif.

16. Dispositif suivant l'une des revendications 9 à 15, un dispositif d'affichage (8) étant prévu pour la représentation des positions déterminées de l'objet (11) dans le système de coordonnées de navigation.

17. Dispositif suivant l'une des revendications 9 à 16, un dispositif (5) de réalisation d'un procédé de résonance magnétique nucléaire, un dispositif de réalisation d'un procédé de tomographie assistée par ordinateur et/ou un dispositif (7) d'acquisition de lumière, en particulier de signaux infrarouges, de signaux radio ou de signaux sonores étant prévus pour l'acquisition de la position dans le système de coordonnées d'acquisition et/ou dans le système de coordonnées de navigation.

18. Dispositif suivant l'une des revendications 9 à 17, au moins un des dispositifs d'acquisition (5, 7) étant prévu pour la détermination de la position dans le système de coordonnées d'acquisition et/ou de navigation étant conçu de façon à être mobile.
